(19) Europäisches Patentamt
European Patent Office
Office européen des brevets

(11) Veröffentlichungsnummer: **0 320 765 A2**

(12) # EUROPÄISCHE PATENTANMELDUNG

(21) Anmeldenummer: **88120407.7**

(22) Anmeldetag: **07.12.88**

(51) Int. Cl.4: **C07D 231/38 , C07D 231/16 , A01N 43/56**

(30) Priorität: **17.12.87 DE 3742822**

(43) Veröffentlichungstag der Anmeldung: **21.06.89 Patentblatt 89/25**

(84) Benannte Vertragsstaaten: **BE CH DE FR GB IT LI NL**

(71) Anmelder: **BAYER AG**
**D-5090 Leverkusen 1 Bayerwerk(DE)**

(72) Erfinder: **Fürstenwerth, Hauke, Dr.**
**Unterölbach 3a**
**D-5090 Leverkusen 3(DE)**

(54) **Verfahren zur Herstellung von 5-Amino-1-phenyl-4-nitro-pyrazolen.**

(57) Die Erfindung betrifft ein neues Verfahren zur Herstellung von 5-Amino-1-phenyl-4-nitropyrazolen, dadurch gekennzeichnet, daß man Arylhydrazine der Formel (II)
Ar-NH-$NH_2$ (II)
mit Nitrocyanenaminen der Formel (III)

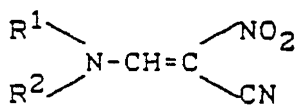

(III)

in Gegenwart eines sauren Reaktionshilfsmittels sowie gegebenenfalls in Gegenwart eines Verdünnungsmittels umsetzt.

EP 0 320 765 A2

## Verfahren zur Herstellung von 5-Amino-1-phenyl-4-nitropyrazolen

Die Erfindung betrifft ein neues Verfahren zur Herstellung von 5-Amino-1-phenyl-4-nitropyrazolen, welche als Herbizide verwendet werden.

Es ist bekannt, daß man 5-Amino-1-phenyl-4-nitropyrazole erhält, wenn man zunächst Arylhydrazine mit 2-Chloracrylnitril cyclisiert, in einer 2. Stufe die so erhältlichen 5-Amino-1-phenyl-pyrazole an der Aminogruppe acyliert, dann in einer 3. Stufe nitriert und schließlich in einer 4. Stufe die Acylschutzgruppe an der Aminofunktion in 5-Stellung des Pyrazolringes wieder abspaltet (vgl. z. B. EP-A 154 115 oder EP-A 224 831).

Der Nachteil des vorbekannten Verfahrens liegt in der vielstufigen Reaktionsführung, die von Aufwand- und Ausbeutebetrachtungen her als unökonomisch auzusehen ist.

Es wurde nun gefunden, daß man 5-Amino-1-phenyl-4-nitropyrazole der allgemeinen Formel (I),

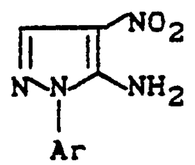 (I)

in welcher
Ar für gegebenenfalls substituiertes Phenyl steht, erhält, wenn man Arylhydrazine der Formel (II),

Ar - NH - $NH_2$ (II)

in welcher
Ar die oben angegebene Bedeutung hat,
mit Nitrocyanenaminen der Formel (III),

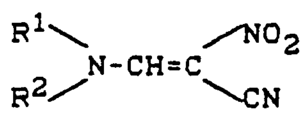 (III)

in welcher
$R^1$ und $R^2$ unabhängig voneinander jeweils für Wasserstoff, Alkyl, Cycloalkyl oder für jeweils gegebenenfalls substituiertes Aralkyl, Aryl oder Heteroaryl stehen oder gemeinsam mit dem Stickstoffatom, an welches sie gebunden sind, für einen gegebenenfalls substituierten Heterocyclus stehen, der gegebenenfalls weitere Heteroatome enthalten kann,
in Gegenwart eines sauren Reaktionshilfsmittels sowie gegebenenfalls in Gegenwart eines Verdünnungsmittels umsetzt.

Dabei ist es als ausgesprochen überraschend anzusehen, daß die Nitrocyanenamine der Formel (III) in so glatter Weise mit den Arylhydrazinen der Formel (II) zu den gewünschten Endprodukten der Formel (I) reagieren, da bei der Polyfunktionalität der eingesetzten Ausgangsprodukte ebenso wie der Endprodukte der Formel (I) eher mit dem Auftreten einer Vielzahl von Nebenreaktionen zu rechnen war. Zu erwarten waren beispielsweise Redoxreaktionen zwischen der Nitrogruppe der Nitrocyanenamine und der Hydrazingruppierung der Arylhydrazine, die zum Auftreten nitroser Gase und in der Folge zu einem komplexen Reaktionsgemisch geführt hätten.
Zu erwarten war außerdem, daß sich die ungesättigten Nitroverbindungen der Formel (III) unter den sauren Reaktionsbedingungen zu Hydroxamsäureestern umsetzen würden (vgl. z. B. Houben-Weyl "Methoden der organischen Chemie", Band X, 4, S. 200 oder Deutsches Bundes Patent DBP 868 906 [1953]).

Das erfindungsgemäße Verfahren weist gegenüber dem vorbekannten vielstufigen Herstellungsverfahren eine Reihe von Vorteilen auf. So erhält man die gewünschten Endprodukte der Formel (I) in hohen Ausbeuten in so großer Reinheit, daß in der Regel auf zusätzliche aufwendige Reinigungsoperationen verzichtet werden kann. Als weiterer Vorteil ist zu werten, daß die einstufige Reaktionsführung zu erheblichen Einsparungen von kostenintensiven Ausgangsmaterialien, Hilfsmitteln, Energie- und Abwassermengen führt, was nicht nur unter ökonomischen sondern auch unter ökologischen Aspekten eine deutliche

Verbesserung gegenüber dem Stand der Technik bedeutet.

Die mit Hilfe des erfindungsgemäßen Verfahrens herstellbaren 5-Amino-1-phenyl-4-nitropyrazole sind durch die Formel (I) allgemein definiert. Bevorzugt herstellbar sind Verbindungen der Formel (I), bei welchen

Ar für gegebenenfalls einfach oder mehrfach, gleich oder verschieden substituiertes Phenyl steht, wobei als Substituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_n-R^3$, wobei

$R^3$ für Amino, sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

n für eine Zahl 0, 1 oder 2 steht.

Besonders bevorzugt herstellbar sind Verbindungen der Formel (I), bei welchen

Ar für gegebenenfalls ein- bis fünffach, gleich oder verschieden substituiertes Phenyl steht, wobei als Substituenten jeweils in Frage kommen: Cyano, Nitro, Fluor, Chlor, Brom, Iod, Methyl, Ethyl, n-oder i-Propyl, n-, i-, s- oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_n-R^3$, wobei

$R^3$ für Amino, Methylamino, Ethylamino, Dimethylamino, Diethylamino, Fluordichlormethyl, Difluormethyl, Tetrafluorethyl, Trifluorchlorethyl, Trichlormethyl, Trichlorethyl, Trifluormethyl, Methyl oder Ethyl steht und

n für eine Zahl 0, 1 oder 2 steht.

Ganz besonders bevorzugt herstellbar sind Verbindungen der Formel (I), bei welchen

Ar für gegebenenfalls ein- bis fünffach, gleich oder verschieden substituiertes Phenyl steht, wobei als Substituenten in Frage kommen: Fluor, Chlor, Brom, Methyl, Ethyl, Methoxy, Ethoxy, Trifluormethyl, Trifluormethoxy, Trifluormethylthio, Trifluormethylsulfinyl oder Trifluormethylsulfonyl.

Verwendet man beispielsweise 2,6-Dichlor-4-trifluormethylphenylhydrazin und 2-Nitro-3-(4-morpholinyl)-acrylnitril als Ausgangsstoffe, so läßt sich der Reaktionsablauf des erfindungsgemäßen Verfahrens durch das folgende Formelschema darstellen:

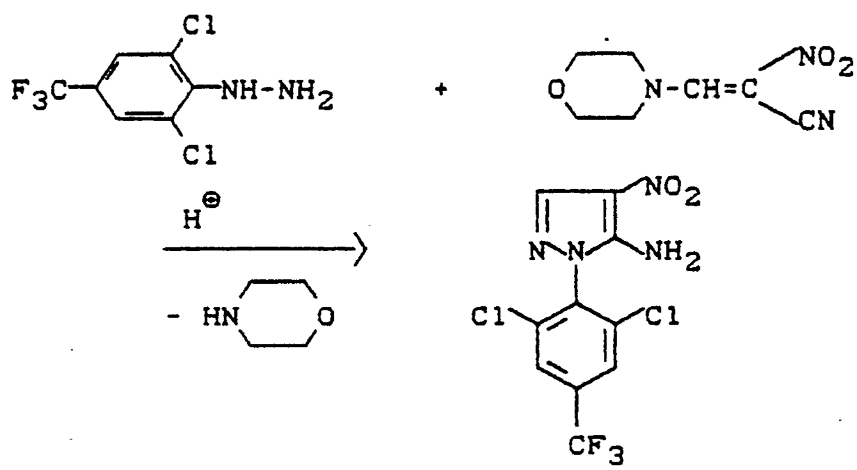

Die zur Durchführung des erfindungsgemäßen Verfahrens als Ausgangsstoffe benötigten Arylhydrazine sind durch die Formel (II) allgemein definiert. In dieser Formel (II) steht Ar vorzugsweise für diejenigen Reste, die bereits im Zusammenhang mit der Beschreibung der erfindungsgemäß herstellbaren Stoffe der Formel (I) als bevorzugt für diese Substituenten genannt wurden.

Die Arylhydrazine der Formel (II) sind bekannt oder erhältlich in Analogie zu bekannten Verfahren (vgl. z. B. EP-A 154 115 oder EP-A 224 831 oder EP-A 187 285 oder EP-A 34 945).

Die zur Durchführung des erfindungsgemäßen Verfahrens weiterhin als Ausgangsstoffe benötigten Nitrocyanenamine sind durch die Formel (III) allgemein definiert. In dieser Formel (III) stehen $R^1$ und $R^2$ vorzugsweise unabhängig voneinander für Wasserstoff, geradkettiges oder verzweigtes Alkyl mit 1 bis 4

Kohlenstoffatomen, für Cycloalkyl mit 3 bis 7 Kohlenstoffatomen oder für jeweils gegebenenfalls ein- bis dreifach gleich oder verschieden substituiertes Phenyl oder Benzyl, wobei als Substituenten in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl oder Ethoxycarbonyl, oder $R^1$ und $R^2$ stehen gemeinsam mit dem Stickstoffatom, an welches sie gebunden sind, für einen fünf- oder sechsgliedrigen gesättigten Heterocyclus, der gegebenenfalls weitere Heteroatome wie insbesondere Stickstoff, Sauerstoff oder Schwefel enhalten kann. $R^1$ und $R^2$ stehen besonders bevorzugt unabhängig voneinander für Wasserstoff, Methyl, Ethyl, n- oder i-Propyl, n-, i-, s- oder t-Butyl, Cyclohexyl, Phenyl oder Benzyl oder gemeinsam mit dem Stickstoffatom, an welches sie gebunden sind, für einen 1-Pyrrolidinylrest, einen 1-Piperidinylrest oder einen 4-Morpholinylrest.

Die Nitrocyanenamine der Formel (III) sind ebenfalls bekannt oder erhältlich in Analogie zu bekannten Verfahren (vgl. z. B. Amer. chem. Journal 29, 253 - 274 [1903]; J. org. Chem. 40, 955 [1979]; Chem. Ber. 114, 3471 [1981]).

Das erfindungsgemäße Verfahren wird in Gegenwart eines sauren Reaktionshilfsmittels durchgeführt. Als solche können übliche Protonen- oder Lewis-Säuren verwendet werden. Mit besonderem Vorzug verwendet man anorganische Mineralsäuren wie Salzsäure oder Schwefelsäure, aliphatische oder aromatische Carbon- oder Sulfonsäuren, wie Essigsäure, Methansulfonsäure oder p-Toluolsulfonsäure, Lewis-Säuren, wie Bortrifluorid, Eisentrichlorid, Aluminiumtrichlorid oder Zinkdichlorid oder saure Ionenaustauscher.

Als Verdünnungsmittel zur Durchführung des erfindungsgemäßen Verfahrens kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide oder Sulfone, wie Dimethylsulfoxid oder Tetramethylensulfon (Sulfolan) oder Alkohole wie Methanol, Ethanol, Propanol, Butanol oder Ethylenglykol, bzw. dessen Monomethyl- oder Monoethylether.

Es ist auch möglich das erfindungsgemäße Verfahren ohne Zusatz eines Verdünnungsmittels oder in Gegenwart eines entsprechenden Überschusses an saurem Reaktionshilfsmittel, welches gleichzeitig als Verdünnungsmittels dient durchzuführen.

Die Reaktionstemperaturen können bei der Durchführung des erfindungsgemäßen Verfahrens in einem größeren Bereicht variiert werden. Im allgemeinen arbeitet man bei Temperaturen zwischen 20 °C und 150 °C, vorzugsweise bei Temperaturen zwischen 80 °C und 100 °C.

Zur Durchführung des erfindungsgemäßen Verfahrens setzt man pro Mol an Arylhydrazin der Formel (II) im allgemeinen 1.0 bis 5.0 Mol, vorzugsweise 1.0 bis 2.0 Mol an Nitrocyanenamin der Formel (III) und 1.0 bis 20.0 Mol, vorzugsweise 1.0 bis 10.0 Mol an saurem Reaktionshilfsmittel ein.
Die Reaktionsdurchführung, Aufarbeitung und Isolierung der Reaktionsprodukte erfolgt nach allgemein üblichen Methoden, beispielsweise durch Entfernen des organischen Verdünnungsmittels, Ausfällen des Reaktionsproduktes in Wasser, Absaugen und Trocknen des so erhältlichen Produktes.

Die mit Hilfe des erfindungsgemäßen Verfahrens herstellbaren 5-Amino-1-phenyl-4-nitropyrazole sind bekannte Herbizide (vgl. z.B. EP-A 154 155 oder EP-A 224 831).

## Herstellungsbeispiele

### Beispiel 1

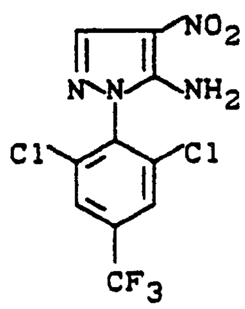

9,2 g (0.05 Mol) 2-Nitro-3-(4-morpholinyl)-acrylnitril, 12,3 g (0.05 Mol) 2,6-Dichlor-4-trifluormethylphenylhydrazin und 10 ml 30 %ige wäßrige Salzsäure in 50 ml Ethanol werden 4 Stunden auf Rückflußtemperatur erhitzt, abgekühlt und im Vakuum eingeengt. Der Rückstand wird mit 100 ml Wasser verrührt, der so erhältliche Niederschlag abgesaugt, mit wasser gewaschen und getrocknet.

Man erhält 15,5 g (90 % der Theorie) an 5-Amino-4-nitro-1-(2,6-dichlor-4-trifluormethylphenyl)-pyrazol vom Schmelzpunkt 190 °C.

Beispiel 2

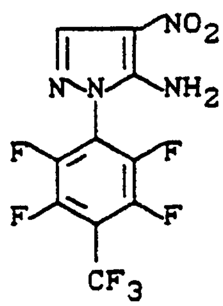

11,2 g (0.05 Mol) 2-Nitro-3-(N-ethyl-cyclohexylamino)-acrylnitril und 12 g (0.05 Mol) 2,3,5,6-tetrafluor-4-trifluormethylphenylhydrazin und 3 ml konzentrierte Schwefelsäure in 100 ml Methanol werden 4 Stunden auf Rückflußtemperatur erhitzt, abgekühlt und portionsweise in 500 ml Wasser gegeben. Die resultierende Suspension wird mit wäßriger Natronlauge auf pH 4 - 5 eingestellt, der ausgefallene Niederschlag abgesaugt, mit Wasser gewaschen und getrocknet.

Man erhält 14 g (81 % der Theorie) an 5-Amino-4-nitro-1-(2,3,5,6-tetrafluor-4-trifluormethylphenyl)-pyrazol vom Schmelzpunkt 122 °C.

In entsprechender Weise erhält man Beispiel 3:

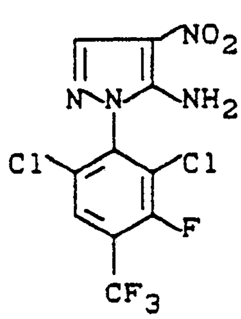

Fp. 147 - 157 °C

Beispiel 4

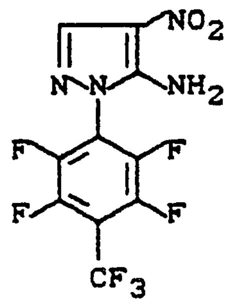

(I)

6,4 g (0,05 Mol) 2-Nitro-3-methylamino-acrylnitril und 12 g (0,05 Mol) 2,3,5,6-tetrafluor-4-trifluormethylphenylhydrazin und 3 ml konzentrierte Schwefelsäure in 100 ml Methanol werden 4 Stunden auf Rückflußtemperatur erhitzt, abgekühlt und portionsweise in 500 ml Wasser gegeben. Die resultierende Suspension wird mit wäßriger Natronlauge auf pH 4 - 5 eingestellt, der ausgefallene Niederschlag abgesaugt, mit Wasser gewaschen und getrocknet.

Man erhält 14 g (81 % der Theorie) an 5-Amino-4-nitro-1-(2,3,5,6-tetrafluor-4-trifluormethylphenyl)-pyrazol vom Schmelzpunkt 122° C.

## Ansprüche

1. Verfahren zur Herstellung von 5-Amino-1-phenyl-4-nitropyrazolen der Formel (I),

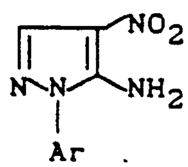

(I)

in welcher
Ar für gegebenenfalls substituiertes Phenyl steht,
dadurch gekennzeichnet, daß man Arylhydrazine der Formel (II),
$Ar - NH - NH_2$ (II)
in welcher
Ar die oben angegebene Bedeutung hat,
mit Nitrocyanenaminen der Formel (III),

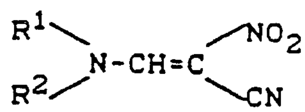

(III)

in welcher
$R^1$ und $R^2$ unabhängig voneinander jeweils für Wasserstoff, Alkyl, Cycloalkyl oder für jeweils gegebenenfalls substituiertes Aralkyl, Aryl oder Heteroaryl stehen oder gemeinsam mit dem Stickstoffatom, an welches sie gebunden sind, für einen gegebenenfalls substituierten Heterocyclus stehen, der gegebenenfalls weitere Heteroatome enthalten kann,
in Gegenwart eines sauren Reaktionshilfsmittels sowie gegebenenfalls in Gegenwart eines Verdünnungsmittels umsetzt.

2. Verfahren nach Anspruch 1, dadurch gekennzeichnet, daß man die Umsetzung bei Temperaturen zwischen 20° C und 150° C durchführt.

3. Verfahren nach Anspruch 2, dadurch gekenzeichnet, daß man die Umsetzung bei Temperaturen zwischen 80° C und 100° C durchführt.

4. Verfahren nach Anspruch 1, dadurch gekennzeichnet, daß man pro Mol an Arylhydrazin der Formel (II) 1 bis 5 Mol an Nitrocyanenamin der Formel (III) und 1 bis 20 Mol an saurem Reaktionshilfsmittel einsetzt.

5. Verfahren nach Anspruch 4, dadurch gekennzeichnet, daß man pro Mol an Arylhydrazin der Formel (II) 1 bis 2 Mol an Nitrocyanenamin der Formel (III) und 1 bis 10 Mol an saurem Reaktionshilfsmittel einsetzt.

6. Verfahren nach Anspruch 1, dadurch gekennzeichnet, daß man Protonen- oder Lewis-Säuren oder saure Ionenaustauscher als saures Reaktionshilfmittel einsetzt.

7. Verfahren nach Anspruch 6, dadurch gekennzeichnet, daß man anorganische Mineralsäuren, aliphatische oder aromatische Carbon- oder Sulfonsäuren als Protonensäuren einsetzt.

8. Verfahren nach Anspruch 6, dadurch gekennzeichnet, daß man Salzsäure, Schwefelsäure, Essigsäure, Methansulfonsäure, p-Toluolsulfonsäure, Bortrifluorid, Eisentrichlorid, Aluminiumtrichlorid oder Zinkdichlorid verwendet.